# EUROPEAN PATENT APPLICATION

(11) **EP 0 776 673 A1**
(43) Date of publication of application: **04.06.1997**
(21) Application number: 96203322.1
(22) Date of filing: 26.11.1996
(51) Int. Cl.: A61M 25/01, A61M 25/00

(54) **Catheter guidable by means of flow**

(30) Priority: 28.11.1995 NL 1001763
(71) Applicant: Cordis Corporation, Miami Lakes Florida 33014 (US)
(72) Inventor: Mous, Frans, 9302 HP Drachten (NL)
(74) Representative: 't Jong, Bastiaan Jacobus

(57) **Abstract**

The invention relates to a catheter comprising a tube-like basic body with a proximal and a distal end, a connecting member arranged to the proximal end, and a tube-like end-section at the distal end which is pliable to such an extent, that it does not retain its shape.
As a result this tube-like end-section is guided and carried along by the surrounding flow, in particular, when the catheter has been introduced into the vascular system of a patient, by the flow of the surrounding blood.
Suitable materials of which to make the pliable end-section are for instance LDPE or PVC plasticized with PUR with a coating of PTFE.
The end-section may comprise a marking element and/or filling material as a result of which this end-section becomes visible when using X-rays and/or under NMR conditions.

## Description

The invention relates to a catheter comprising a tube-like basic body with a proximal and a distal end. A connecting member has been arranged to the proximal end and a tube-like end-section to the distal end.

Such catheters are generally known. They are introduced into a patient and the tube-like end-section is advanced to a site where an investigation or remedial treatment is to be carried out. With the known catheter the end-section may be preformed, so that it will take up more or less automatically a desired position in the area to be investigated or treated.

With the catheter according to the invention the tube-like end-section has been made pliable to such a degree that it does not retain its shape. As a result this tube-like end-section is advanced and carried along by the surrounding flow, in particular, if the catheter has been introduced into the vascular system of a patient, by the flow of the surrounding blood. Consequently the catheter will more or less automatically course down the bloodstream in order to finish up in the desired position.

The catheter according to the invention is particularly suitable for introduction into very small and tortuous blood vessels as can be found in the brain of a patient. In order to ensure that the catheter can be controlled properly, the measure as set out in claim 2 is preferably employed.

The relatively distal second section of the end-section can be given the desired greater pliability by means of a suitable choice of material. Additionally or instead of that, the measure as set out in claim 3 can be employed in an advantageous manner. This measure has the added advantage that the outside diameter of the end-section is adjusted as it were to the diameter of the blood vessels inside of which it is to be received. The diameter of a lumen extending through the catheter can consequently be given, as far as inside the end-section, a diameter which is as large as possible, as a result of which the flow resistance to which a liquid flowing through the lumen is subjected, can remain as small as possible.

Furthermore, this relatively limited flow resistance is advantageous when positioning the catheter. When doing so, short liquid pulses under high pressure can be injected into the catheter. As a result of the reactive force occurring at the end of the catheter, the distal end can be controlled in order to be positioned in the desired blood vessel. Due to the relatively limited flow resistance, a good manoeuvrability can be achieved in this way.

A very suitable embodiment is characterised in claim 4. In general, the second, most pliable section only needs to be a quarter of the length of the first section of the end-section in order to function well. Preferably the measure as set out in claim 5 is employed. The catheter according to the invention can for instance be used for embolization treatment. An embolization medium such as cyano acrylate or ethanol may be supplied through the lumen. For such treatment a catheter with the measure as set out in claim 6 is preferably employed. As a result it will be ensured that, in the event of the catheter bursting, for instance as a result of incorrect use, this will occur in the second, most pliable section which is located closest to the distal end. In the event of such a calamity, the embolization medium cannot finish up in the more proximal section of the blood vessel through which the catheter has been introduced, with all the attendant risks.

For such an application the embodiment of the catheter according to the invention as characterised in claim 7 is very suitable.

A suitable pliability is achieved when the measure as set out in claim 8 is employed.

A suitable material of which to make the catheter according to the invention is characterised in claim 9. Such a polyethylene-elastomer has a very dense molecular distribution so that is can be used for medical purposes. Furthermore, this LDPE is inert to the action of the chemical substances to be used in conjunction with the catheter.

Another suitable embodiment is characterised in claim 10. In order to render this embodiment more suitable for use with the substances mentioned, in particular cyano acrylate, the measure as set out in claim 11 is preferably employed. The cyano acrylate can in that case not combine with the material of which the pliable end-section has been made.

Yet another suitable embodiment is characterised in claim 12. SBSE can have such a composition that it has rubbery properties, which is most suitable for the intended application.

In order to achieve optimum mechanical properties the measure as set out in claim 13 can be employed in a favourable manner. The different plastic materials can be received in the end-section by means of multilayer co-extrusion or strip-shaped extrusion.

In order to be able to introduce the catheter properly into a patient, the measure as set out in claim 14 is preferable employed. The relatively stiff basic body will extent through relatively wide blood vessels and the pliable end-section will extend through the narrower blood vessels. For the introduction a very thin guide wire, which stiffens the pliable end-section, can be used until it reaches the narrow blood vessels. Next the pliable end-section can be advanced further into the narrower blood vessels by means of the surrounding flow. The intermediate section - of which there is at least one - ensures a gradual
transition in pliability from the relatively stiff basic body to the very pliable end-section. When the measure as set out in claim 15 is employed, the required stiffness of the proximal section of the catheter can be achieved in a suitable manner. Additionally, a suitable embodiment is characterised in claim 16.

In order to be able to monitor the progress of the relatively distal end of the pliable end-section in a catheterization laboratory, the measure as set out in claim 17 is preferably employed. In addition, the measure as set out in claim 18 is preferably employed. With the application for embolization treatment described above, it is prevented in this way that the marking element is glued to the tissue.

The measure as set out in claim 19 can be employed in a suitable manner.

The invention will be explained in greater detail with reference to the attached drawings, illustrating a catheter according to the invention in the vicinity of the heart of a patient.
Figure 1 illustrates a first phase of the introduction of a catheter according to the invention; and
figure 2 illustrates a final phase of this procedure;
figure 3 illustrates an application of the catheter according to the invention which is particularly suitable;
figure 4 shows the end-section of the catheter employed in figure 3.

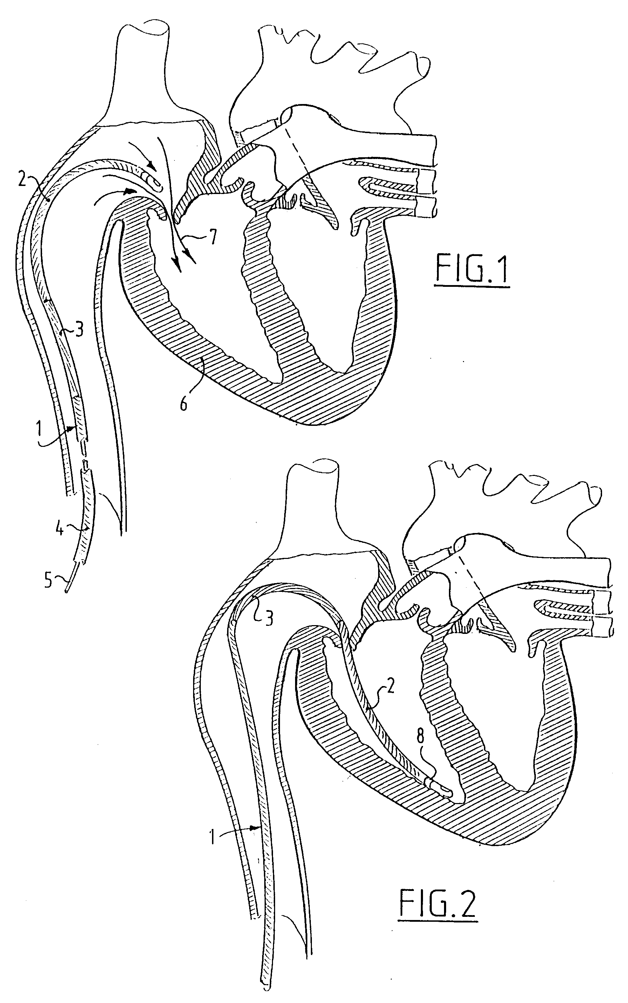

In figures 1 and 2 the distal end of a catheter 1 according to the invention is shown. It comprises a tube-like basic body 4, to which, via an intermediate section 3, an end-section 2 has been arranged. A lumen extends through the basic body 4, the intermediate section 3 and the end-section 2, inside of which a guide wire 5 can be received and through which for instance a diagnostic or treatment medium can be supplied to the distal end.

The basic body 4 comprises at the proximal end, which has not been illustrated here, a connecting piece known as such, like for instance a Luer-connector.

The end-section 2 of the catheter 1 according to the invention has been made pliable to such an extent that it does not retain its shape. As a result this end-section 2 is carried along and guided by the surrounding flow.

With the example of the embodiment illustrated in the figures, the end-section 2 of the catheter 1 is to be positioned in a chamber of the heart 6. As has been indicated with the arrows 7, blood enters on expansion of the chamber. The very pliable end-section 2 is carried along by this flow and as a result finishes up inside the chamber of the heart 6, as has been illustrated in figure 2. A guide wire 5 can be used in order to introduce the catheter and in particular to guide the pliable end-section 2 to the vicinity of the target-position. Only during the last phase of introducing the catheter the pliable end-section 2 is released, as a result of which it can be carried along with the surrounding flow.

Close to its relatively distal end, the end-section 2 has been provided with a marking ring 8, which makes the distal end visible on an X-ray screen. The material of which the end-section 2 has been made may also comprise filling materials, as a result of which this end-section becomes visible in its entirety on an X-ray screen and/or under NMR-conditions.

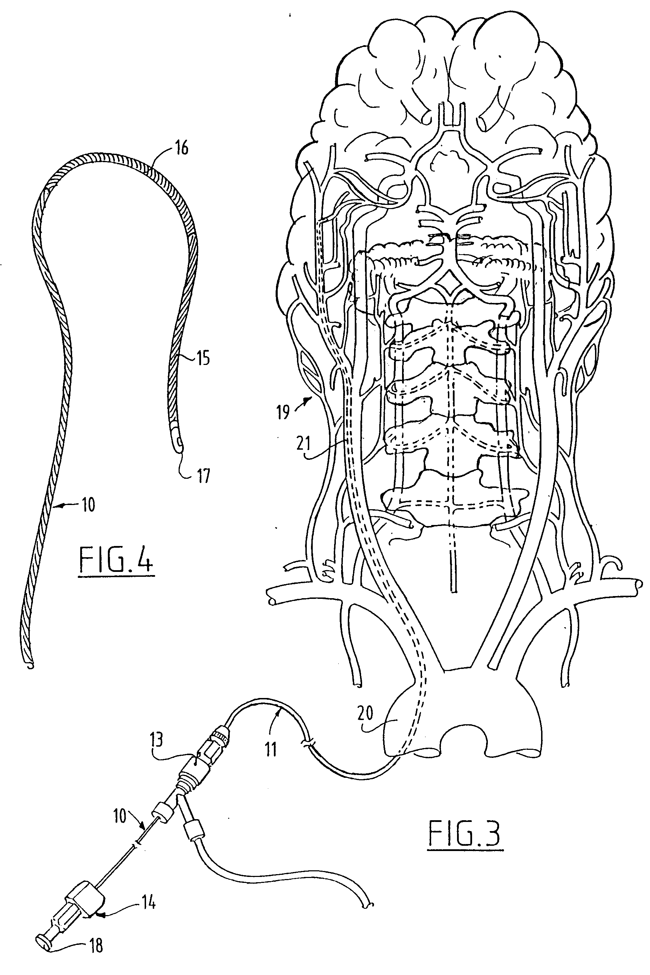

Although an embodiment of the catheter according to the invention can be used in the way illustrated in the figures 1 and 2, the advantages of the invention are done real justice with neuro-radiological applications. In that case the distal end of a catheter with a very small diameter, for instance of the order of 0.5 mm, is introduced into the vascular system of the head. This has been illustrated schematically in figure 3.

The catheter 10 shown in that figure is introduced through a haemostatic device 13 of a guiding catheter 11. Prior to that, the guiding catheter 11 has been advanced via the femoral artery and the aorta 20 of the patient into the carotid artery 21. From there the pliable end-section 15 of the catheter can be carried along with the blood flow as far as the very small blood vessels of the brain. The vascular system of the head as a whole has been referred to with the reference number 19.

As has been mentioned before, the catheter 10 has a very pliable end-section 15 which changes, via a somewhat less pliable section 16, into the remaining part of the basic body. A lumen extends through the catheter which ends at the distal end in an end-opening 17. At the proximal end the lumen is connected to a connection 18 of the connecting member 14. Once the distal end-section of the catheter has arrived at the desired location, part of the vascular system in the vicinity of the end-section of the catheter can be visualized in a catheterization laboratory by means of supplying a contrast medium via the connection 18.

Although it has not been illustrated in detail in the figures, the catheter according to the invention may be made up, from the proximal towards the distal end, of a number of sections of decreasing stiffness. In particular the pliable end-section may be made up of two sections, whereby the length of the relatively distal section is about one quarter of that of the relatively proximal section.

Additionally, the end-section has been made in such a way that the diameter decreases towards the distal end. This is achieved by giving the most pliable end a smaller diameter. The decreasing diameter and decreasing stiffness towards the distal end ensure a good manoeuvrability of the catheter-end-section.

As the catheter, or at least its pliable end-section, is carried along by the blood flow, the advantage of a very a-traumatic treatment is achieved, which is of the utmost importance when the vascular system is weak. The risk of damaging the vascular wall at a weak spot, with the concomitant serious threat of a cerebral haemorrhage, is prevented to a significant degree with the catheter according to the invention. In addition, as a result of the a-traumatic action of the catheter according to the invention, the occurrence of spasms during the procedure is avoided in a favourable manner as well.

Due to the small diameter of a catheter for such applications, a desired great pliability can easily be achieved, as a result of which the catheter-end-section can be advanced into the very small and tortuous blood vessels of the brain of a patient. In conjunction with a small diameter, the end-section has a relatively large outside surface area, so that the impact of the surrounding flow on it can be relatively large as a result of which the end-section is carried along easily into the small blood vessels.

In the case of that embodiment the catheter-end-section can be advanced into the very small and tortuous blood vessels of the brain of a patient. As a result of the small diameter of such an end-section a desired great pliability can be achieved very easily. In addition, with a decreasing diameter, such an end-section has a relatively large outside surface area, so that the impact of the surrounding flow on it can be relatively large.

Although the invention has been explained above with reference to an application-example in the vicinity of the heart and the brain, the invention is obviously not limited to catheters for use in these circumstances. Also for use in the vicinity of other organs, such as the liver and the kidneys, the invention is very suitable.

## Claims

1. Catheter comprising a tube-like basic body with a proximal and a distal end, a connecting member arranged to the proximal end and a tube-like end-section at the distal end which is pliable to such an extent that it does not retain its shape.

2. Catheter as claimed in claim 1, wherein the end-section comprises a relatively proximal first section and a relatively distal second section, wherein the second section is more pliable than the first section.

3. Catheter as claimed in claim 2, wherein the second section has a smaller diameter than the first section.

4. Catheter as claimed in claim 3, wherein the first section has a diameter of the order of 0.6 - 0.9 mm and the second section a diameter of the order of 0.4 - 0.7 mm.

5. Catheter as claimed in one of the claims 2 - 4, wherein the first section has a length of the order of 10 - 30 cm and the second section a length of the order of 2 - 7 cm.

6. Catheter as claimed in one of the claims 2 - 5, wherein the first section has a higher bursting pressure than the second section.

7. Catheter as claimed in claim 6, wherein the first section has a bursting pressure of at least 28 bar (200 psi) and the second section a bursting pressure of at least 27 bar (190 psi).

8. Catheter as claimed in one of the previous claims, wherein the end-section has an elastic modulus not exceeding 10 N/mm².

9. Catheter as claimed in one of the previous claims, wherein the end-section comprises polyethylene-elastomer (LDPE).

10. Catheter as claimed in one of the claims, wherein the end-section comprises polyvinyl chloride (PVC) plasticized with polyurethane (PUR).

11. Catheter as claimed in claim 10, wherein at least around the relatively distal end of the pliable end-section a coating of polytetrafluoroethene (PTFE) has been applied.

12. Catheter as claimed in one of the claims, wherein the end-section comprises a styrene ethyl block styrene polymer (SBSE).

13. Catheter as claimed in one of the claims 10 -12, wherein the end-section comprises in cross-sectional direction a number of separate areas containing different plastic materials.

14. Catheter as claimed in one of the previous claims, wherein the basic body is relatively stiff and comprises in between the basic body and the pliable end-section at least one intermediate section with a stiffness in between that of the adjacent sections.

15. Catheter as claimed in claim 14, wherein a proximal end-section of the basic body has been reinforced with a tube-like stiffening body arranged around it.

16. Catheter as claimed in claim 15, wherein the length of the stiffening body is 20 - 60 % of that of the catheter.

17. Catheter as claimed in one of the previous claims, wherein the pliable end-section at the relatively distal end has been provided with a marking element visible when using X-rays.

18. Catheter as claimed in claim 17, wherein the marking element has been embedded in the material of which the end-section has been made.

19. Catheter as claimed in one of the previous claims, wherein the material of which the end-section has been made comprises a filling material which renders the end-section visible when using X-rays and/or under NMR-conditions.
